(19) Europäisches Patentamt
European Patent Office
Office européen des brevets

(11) Publication number: 0 266 450 B1

(12) EUROPEAN PATENT SPECIFICATION

(45) Date of publication of patent specification: **15.01.92**

(51) Int. Cl.⁵: C07D 493/04, //(C07D493/04, 307:00,307:00)

(21) Application number: **86115333.6**

(22) Date of filing: **05.11.86**

(54) **Process for the selective preparation of isosorbide-2-mononitrate from isosorbide-2,5-dinitrate.**

(43) Date of publication of application:
**11.05.88 Bulletin 88/19**

(45) Publication of the grant of the patent:
**15.01.92 Bulletin 92/03**

(84) Designated Contracting States:
**CH DE ES FR GB LI**

(56) References cited:
**EP-A- 0 059 664**
**EP-A- 0 067 964**
**US-A- 4 065 488**

(73) Proprietor: **CONSIGLIO NAZIONALE DELLE RICERCHE**
**Piazzale Aldo Moro, 7**
**I-00198 Roma(IT)**

Proprietor: **DINAMITE DIPHARMA Spa**
**Viale Duodo 3**
**I-33100 Udine(IT)**

(72) Inventor: **Camera, Ettore**
**via Paternoldi 11**
**Gorizia(IT)**
Inventor: **De Lucchi, Ottorino**
**via Facciolati 40/1B**
**Padova(IT)**
Inventor: **Filipuzzi, Fabiola**
**via S. Maria Iconia 16/B15**
**Padova(IT)**
Inventor: **Modena, Giorgio**
**via S. Giovanni Da Verdara 135**
**Padova(IT)**

(74) Representative: **Gervasi, Gemma, Dr. et al**
**NOTARBARTOLO & GERVASI Srl 33, Viale Bianca Maria**
**I-20122 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 266 450 B1

## Description

This invention relates to a new process for the preparation of isosorbide-2-mononitrate (I):

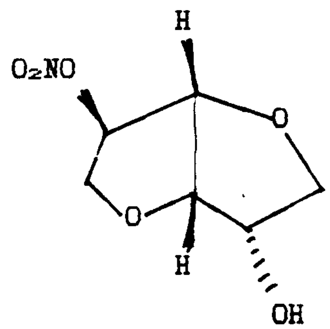

(I)

More particularly, the invention relates to a process for the selective preparation of isosorbide-2-mononitrate (I) from isosorbide-2,5-dinitrate (II):

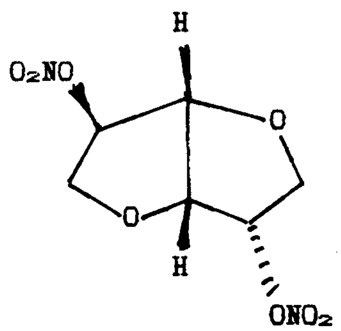

(II)

Isosorbide-2-mononitrate (I) is of considerable pharmaceutical importance in that it acts as a non-specific smooth muscle relaxant, and particularly as a coronary vasodilator (J. Pharmacol. Exp. Ther., 180, 732-742, 1972; Nuov. Presse Med., Tile 9, 2424-2427, 1980).

Isosorbide-5-mononitrate (III)

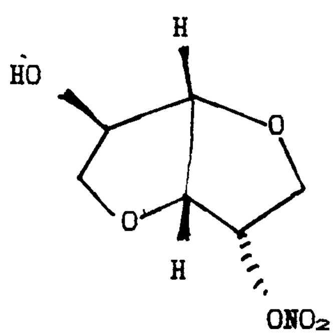

(III)

also presents analogous interesting characteristics from the pharmacological viewpoint. However, whereas for compound (I) the half-life is 1.8 hours, for compound (III) the half-life is 4.2 hours.

Because of this fundamental difference in the pharmaceutical application of the two compounds, it is necessary when preparing them from isosorbide-2,5-dinitrate (II) to operate in such a manner as to selectively obtain the one or other of the two isomers (I) and (III).

Some processes are known for preparing isosorbide mononitrate.

However, only one process for the selective preparation of isosorbide-2-mononitrate (I) is known (German patent No. 2,903,983). In this process, tetrabutylammonium nitrite and isomannide-2-triflate are used as reagents, these being of laborious preparation and high cost. This is therefore a process which is

not suitable for industrial application.

Other known processes for preparing isosorbide-2-mononitrate use isosorbide-2,5-dinitrate (II) as their starting substance, but these are non-selective processes, such as the process described in French patent No. 8,103,906, in which isosorbide-2,5-dinitrate (II) is treated with hydrazine hydrate to obtain a mixture of the two isomers (I) and (III). This process aslo has the drawbacks of operating with a reagent which is a known carcinogen, namely hydrazine hydrate, and of being of low yield.

We have now discovered a new process for the preparation of isosorbide-2-mononitrate (I) from isosorbide-2,5-dinitrate, which compared with known processes has remarkable advantages, including:

- the use of easily obtainable low-cost reagents;
- easily attainable operating conditions;
- high selectivity and high yield of the required product;
- ease of application to industrial production.

The process for the preparation of isosorbide-2-mononitrate according to the invention is characterised in that isosorbide-2,5-dinitrate is treated, in a reaction medium consisting of an organic solvent and water, with salts of metals of low oxidation state, to obtain isosorbide-2-monoitrate (I) with high selectivity.

These and further characteristics and advantages of the process according to the present invention will be more apparent from the non-limiting description of preferred methods of implementing the process, given hereinafter for illustrative purposes.

The reaction medium preferably consists of ethyl alcohol and water in a volume ratio of between 50/50 and 80/20, but other organic solvents can be used instead of the ethyl alcohol, such as methyl alcohol, propyl alcohol, acetonitrile, acetic acid, ethylene glycol, dioxane, tetrahydrofuran etc.

The inorganic compounds preferably used are ferrous sulphate and cuprous chloride. However, use can also be made of other saline compounds of Cu(I) and Fe(II), or of other metals such as Co(II), Cr(II), Mn(II), Pb(II) and Sn(II), and in general salts of metal ions which can act as reducing agents thus attaining higher oxidation states.

The isosorbide-2,5-dinitrate is dissolved in the reaction medium under agitation at ambient temperature, and the salt is then added under agitation.

The temperature is brought to between 0°C and the boiling point of the mixture under reflux, and the reaction is continued under agitation for a time of between 2 and 48 hours.

The ratio of moles of isosorbide-2,5-dinitrate to gram equivalents of reducing ions used for the reaction is preferably between 1 and 10.

On termination of the reaction, the suspension obtained is cooled and then filtered. The solution is evaporated to dryness under vacuum, and the residue dissolved in a small quantity of $CH_2Cl_2$. If necessary, any insoluble residues are filtered off, after which a seeding crystal of isosorbide-2-mononitrate is added to the solution to crystallise the product isosorbide-2-mononitrate with high purity.

If unreacted isosorbide-2,5-dinitrate is present in the reaction mixture after the preparation, it can be easily recycled to the next preparation.

The following examples are given as non-limiting illustration of the characteristics of the process according to the invention.

EXAMPLE 1

50 ml of an aqueous ethanol solution with an ethanol/water volume ratio of 70/30 are fed into a 100 ml laboratory flask fitted with a thermometer and agitator. 1 g (4.2 mmoles) of isosorbide-2,5-dinitrate is then added and is dissolved under agitation. Finally, 6 g (21.6 mmoles) of $FeSO_4.7H_2O$ heptahydrate are added. The mixture is heated and kept boiling under reflux for 24 hours under agitation. On termination of the reaction, the suspension obtained is cooled to 20°C and filtered. The solution is evaporated under vacuum and 2 ml of $Ch_2Cl_2$ are then added. A seeding crystal of isosorbide-2-mononitrate is then added to crystallise 0.65 g (3.4 mmoles) of isosorbide-2-mononitrate (yield 81%) which on GLC analysis is found to be of 98% purity. M.P. = 55°C (53°C in the literature).

EXAMPLE 2

10 ml of an aqueous ethanol solution with an ethanol/water volume ratio of 70/30 are fed into a laboratory flask as in Example 1. 1 g (4.2 mmoles) of isosorbide-2,5-dinitrate is then added and is dissolved under agitation. Finally, 3 g (30 mmoles) of cuprous chloride are added. The mixture is heated and kept boiling under reflux for 12 hours under agitation. After this reaction time, the mixture is analysed by gas chromatography and the following results are found: isosorbide 15%, isosorbide-2-nitrate 45%, isosorbide-5-nitrate 6.8% and unreacted isosorbide-2,5-dinitrate 33.2%.

## Claims

1. A selective process for the preparation of isosorbide-2-mononitrate

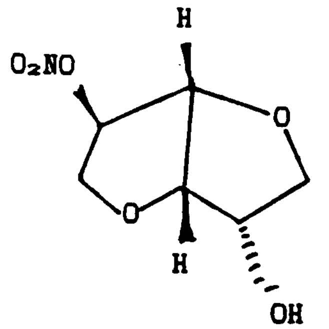

(I)

from isosorbide-2,5-dinitrate

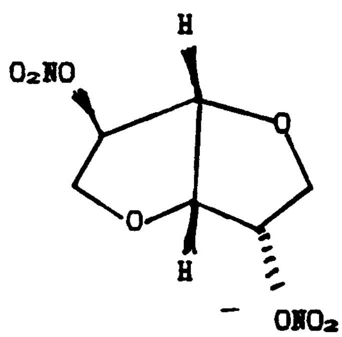

(II)

characterised in that the isosorbide-2,5-dinitrate (II) is treated, in a reaction medium consisting of water and an organic solvent miscible with water and not subjected to reduction in the reaction conditions, with salts of metals of low oxidation state, selected from Cu(I), Fe(II), Co(II), Mn(II), Pb(II), Sn(II) and Cr(II).

2. A process as claimed in claim 1, characterised in that said salts of metals of low oxidation state are salts of Fe(II) or of Cu(I).

3. A process as claimed in claim 1, characterised in that said treatment of isosorbide-2,5-dinitrate is carried out at a temperature of between 0 ° C and the reflux temperature, under agitation.

4. A process as claimed in claim 1, characterised in that said treatment of isosorbide-2,5-dinitrate is carried out for a time of between 2 and 48 hours.

5. A process as claimed in claim 1, characterised in that the ratio of moles of isosorbide-2,5-dinitrate (II) to gram equivalents of the salts of metals of low oxidation state as defined in claim 1, is between 1 and 10.

## Revendications

1. Procédé de préparation sélective de l'isosorbide-2-mononitrate

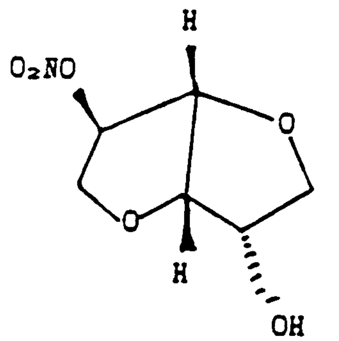

(I)

à partir de l'isosorbide-2,5-dinitrate

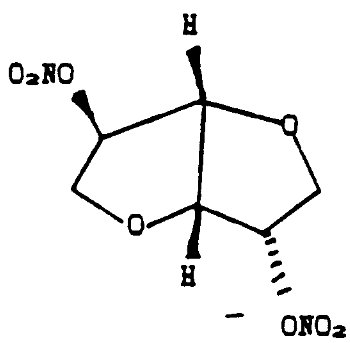

(II)

caractérisé en ce que l'on traite l'isosorbide-2,5-dinitrate (II) dans un milieu réactionnel, constitué par de l'eau et un solvant organique miscible avec l'eau et non soumis à une réduction dans les conditions de la réaction, avec des sels de métaux à faible état d'oxydation, choisis parmi Cu(I), Fe(II), Co(II), Mn(II), Pb(II), Sn(II) et Cr(II).

2. Procédé tel que revendiqué dans la revendication 1, caractérisé en ce que lesdits sels de métaux à faible état d'oxydation sont des sels de Fe(II) ou de Cu(I).

3. Procédé tel que revendiqué dans la revendication 1, caractérisé en ce que le traitement de l'isosorbide-2,5-dinitrate est effectué à une température comprise entre 0 °C et la température de reflux, sous agitation.

4. Procédé tel que revendiqué dans la revendication 1, caractérisé en ce que le traitement de l'isosorbide-2,5-dinitrate est effectué pendant une durée comprise entre 2 heures et 48 heures.

5. Procédé tel que revendiqué dans la revendication 1, caractérisé en ce que le rapport des moles de l'isosorbide-2,5-dinitrate(II) aux équivalents-grammes des sels de métaux à faible état d'oxydation, tels que définis dans la revendication 1, est compris entre 1 et 10.

**Patentansprüche**

1. Selektives Verfahren zur Herstellung von Isosorbid-2-mononitrat

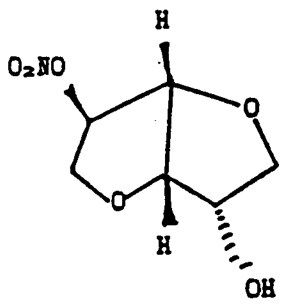

(I)

aus Isosorbid-2,5-dinitrat

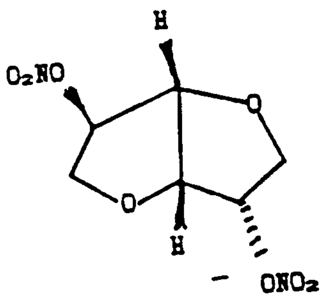

(II)

**dadurch gekennzeichnet,** daß das Isosorbid-2,5-dinitrat (II) in einem Reaktionsmedium bestehend aus Wasser und einem mit Wasser mischbaren organischen Lösungsmittel mit Salzen von Metallen niederer Oxidationsstufe ausgewählt aus Cu(I), Fe(II), Co(II), Mn(II), Pb(II), Sn(II) und Cr(II) behandelt wird und unter den Reaktionsbedingungen nicht einer Reduktion unterliegt.

2. Verfahren nach Anspruch 1, **dadurch gekennzeichnet,** daß die Salze der Metalle mit niederer Oxidationsstufe Salze von Fe(II) oder von Cu(I) sind.

3. Verfahren nach Anspruch 1, **dadurch gekennzeichnet,** daß die Umsetzung von Isosorbid-2,5-dinitrat bei einer Temperatur zwischen 0 °C und der Rückflußtemperatur unter Rühren ausgeführt wird.

4. Verfahren nach Anspruch 1, **dadurch gekennzeichnet,** daß die Umsetzung von Isosorbid-2,5-dinitrat innerhalb von 2 bis 48 Stunden durchgeführt wird.

5. Verfahren nach Anspruch 1, **dadurch gekennzeichnet,** daß das Molverhältnis von Isosorbid-2,5-dinitrat (II) zu den Grammäquivalenten der Metallsalze niederer Oxidationsstufe gemäß Anspruch 1 zwischen 1 und 10 liegt.